# EUROPEAN PATENT APPLICATION

(11) **EP 4 800 103 A1**
(43) Date of publication of application: **02.09.2026**
(21) Application number: 24913526.0
(22) Date of filing: 24.12.2024
(51) Int. Cl.: C12N 9/00, C12N 15/77, C12P 19/32

(54) **NOVEL PHOSPHORIBOSYL AMINOIMIDAZOLE-SUCCINOCARBOXAMIDE SYNTHASE VARIANT AND METHOD FOR PRODUCING PURINE NUCLEOTIDE USING SAME**

(30) Priority: 26.12.2023 KR 20230190928
(71) Applicant: CJ Cheiljedang Corporation, Seoul 04560 (KR)
(72) Inventor: BONG, Hyunju, Seoul 04560 (KR); KWON, Hee Su, Seoul 04560 (KR); LEE, Ji Hye, Seoul 04560 (KR); RHO, Jin Ah, Seoul 04560 (KR); KIM, Doyeon, Seoul 04560 (KR); LEE, Ji Hyun, Seoul 04560 (KR); KIM, Dae Young, Seoul 04560 (KR)
(74) Representative: Meissner Bolte Partnerschaft mbB
(86) International application number: PCT/KR2024/021002
(87) International publication number: WO 2025/143735

(57) **Abstract**

Provided are: a phosphoribosyl aminoimidazole-succinocarboxamide synthase variant; a microorganism comprising the variant; a composition for producing a purine nucleotide, the composition comprising the microorganism; and a method for producing a purine nucleotide, the method comprising a step for culturing the microorganism.

## Description

### [TECHNICAL FIELD]

### Cross-reference to related application(s)

The present disclosure claims the benefit of priority based on Korean Patent Application No. 10-2023-0190928 filed on January 26, 2023, and the entire contents dissclosed in the documents of the corresponding Korean patent application are incorporated as part of the present disclosure.

The present disclosure relates to a novel phosphoribosyl aminoimidazole-succinocarboxamide synthase variant and a method for producing purine nucleotides using the same.

### [BACKGROUND ART]

Purine nucleotides, for example, 5'-inosine monophosphate (hereinafter, IMP), 5'-xanthosine monophosphate (hereinafter, XMP) and 5'-guanosine monophosphate (hereinafter, GMP) are intermediate substances in nucleic acid biosynthetic metabolic pathways, play physiologically important roles in the body, and are widely used in foods, pharmaceuticals, and the like. Specifically, IMP itself imparts a beef taste, and GMP derived from XMP is known to impart a mushroom taste, and both substances are known to enhance the flavor of monosodium glutamate (MSG), and thus are receiving attention as savory nucleic acid-based seasonings.

The biosynthetic pathway for producing purine nucleotides is very complex, and a series of reactions in which various amino acids and cofactors additionally participate occur sequentially. Among them, the reaction from CAIR (5-amino-1-(5-phospho-D-ribosyl)imidazole-4-carboxylate) to SAICAR ((2S)-2-[5-amino-1-(5-phospho-beta-D-ribosyl)imidazole-4-carboxamido]succinate) is catalyzed by phosphoribosyl aminoimidazole-succinocarboxamide synthase (purC). As substrates for the phosphoribosyl aminoimidazole-succinocarboxamide synthase reaction, not only CAIR (5-amino-1-(5-phospho-D-ribosyl)imidazole-4-carboxylate) but also aspartate and ATP are used.

Conventionally, *Corynebacterium* strains in which genes related to purine biosynthesis are enhanced and methods for producing IMP or XMP using the same are known. For example, a *Corynebacterium ammoniagenes* strain in which a phosphoribosyl pyrophosphate amidotransferase enzyme encoded by *purF* is enhanced and a method for producing XMP using the same are disclosed (Korean Patent Publication No. 10-2007-0056491).

### [DISCLOSURE]

### [TECHNICAL PROBLEM]

One embodiment of the present disclosure provides a polypeptide having phosphoribosyl aminoimidazole-succinocarboxamide synthase activity.

The polypeptide may comprise an amino acid sequence in which an amino acid corresponding to the 69th residue in the amino acid sequence of SEQ ID NO: 5 is substituted with another amino acid.

Another embodiment of the present disclosure provides a polynucleotide encoding the polypeptide.

Another embodiment of the present disclosure provides a recombinant vector comprising the polynucleotide.

Another embodiment of the present disclosure provides a microorganism producing purine nucleotides, in which the activity of phosphoribosyl aminoimidazole-succinocarboxamide synthase is enhanced.

Another embodiment of the present disclosure provides a microorganism, comprising at least one selected from the group consisting of the modified polypeptide, a polynucleotide encoding the modified polypeptide, and a vector comprising the polynucleotide.

Another embodiment of the present disclosure provides a method for producing purine nucleotides, comprising culturing the microorganism in a medium.

Another embodiment of the present disclosure provides a composition for producing purine nucleotides, comprising the microorganism.

Another embodiment of the present disclosure provides a use of the microorganism for producing purine nucleotides.

Another embodiment of the present disclosure provides a use of the microorganism for the preparation of a composition for producing purine nucleotides.

### [TECHNICAL SOLUTION]

This will be described in detail as follows. Meanwhile, each description and embodiment disclosed in the present disclosure may be applied to each other description and embodiment. In other words, all combinations of the various elements disclosed in the present disclosure fall within the scope of the present disclosure. In addition, the scope of the present disclosure should not be construed as being limited by the specific descriptions described below. Furthermore, throughout the present disclosure, a number of papers and patent documents are referenced and citations thereof are indicated. The disclosed contents of the cited papers and patent documents are incorporated in the present disclosure by reference in their entirety, thereby more clearly explaining the level of the technical field to which the present disclosure pertains and the contents of the present disclosure.

The present disclosure aims to search for a variant that enhances the activity of phosphoribosyl aminoimidazole-succinocarboxamide synthase, and to provide microorganisms with excellent purine nucleotide production ability by introducing the variant into microorganisms or by preparing microorganisms comprising the variant.

In the present disclosure, it was confirmed that the purine nucleotide production ability is further increased, when an amino acid substitution is introduced at a specific position of phosphoribosyl aminoimidazole-succinocarboxamide synthase.

One embodiment of the present disclosure provides a polypeptide having phosphoribosyl aminoimidazole-succinocarboxamide synthase activity. The polypeptide may be a variant of phosphoribosyl aminoimidazole-succinocarboxamide synthase derived from a microorganism of the genus *Corynebacterium*.

In one specific embodiment, the polypeptide may comprise an amino acid sequence in which an amino acid corresponding to the 69th residue from the N-temrinus in the amino acid sequence of SEQ ID NO: 5 is substituted with another amino acid. As described above, counting amino acids from the N-terminus in the amino acid sequence may refer to counting with methionine (Met, M) translated from a start codon as the first amino acid.

In the present disclosure, the term "phosphoribosyl aminoimidazole-succinocarboxamide synthase (purC)" has activity to catalyze chemical reaction of 5-amino-1-(5-phospho-D-ribosyl)imidazole-4-carboxylate + ATP + L-aspartate → (2S)-2-[5-amino-1-(5-phospho-beta-D-ribosyl)imidazole-4-carboxamido]succinate + ADP + 2 H+ + phosphate. Specifically, the phosphoribosyl aminoimidazole-succinocarboxamide synthase of the presnet disclosure may be used with "purC" interchangeably. In the present disclosure, the sequence of the phosphoribosyl aminoimidazole-succinocarboxamide synthase can be obtained from GenBank of NCBI, which is a publicly known database.

The protein to which a mutation is to be introduced in the present disclosure may be a wild-type protein having phosphoribosyl aminoimidazole-succinocarboxamide synthase activity. Specifically, the phosphoribosyl aminoimidazole-succinocarboxamide synthase to which a mutation is to be introduced may have, comprise, consist of, or essentially consist of the amino acid sequence of SEQ ID NO: 5, but is not limited thereto. In other words, this does not exclude insignificant sequence additions to the N-terminus and/or C-terminus of the amino acid sequence of SEQ ID NO: 5, naturally occurring mutations, or potential mutations thereof (silent mutation), and any protein having the same or corresponding activity as a protein comprising the amino acid sequence of SEQ ID NO: 5 may fall within the scope of the protein to which a mutation is to be introduced in the present disclosure. For example, the protein to which a mutation is to be introduced in the present disclosure may be a protein consisting of an amino acid sequence having 80%, 85%, 90%, 95%, 96%, 97%, 98%, 99%, 99.1%, 99.3%, 99.5%, 99.7%, or 99.9% or more, and less than 100% sequence homology or identity with the amino acid sequence of SEQ ID NO: 5. In addition, an amino acid sequence having such homology or identity and exhibiting an activity corresponding to that of the protein may also be included within the scope of the protein to which a mutation is to be introduced in the present disclosure, even if the protein has an amino acid sequence in which a portion of the sequence is deleted, modified, substituted, or added.

In the present disclosure, the phosphoribosyl aminoimidazole-succinocarboxamide synthase may be derived from a microorganism of the genus *Corynebacterium*, specifically, *Corynebacterium stationis* (*Corynebacterium ammoniagenes*), but is not limited thereto.

In one embodiment of the present disclosure, the amino acid corresponding to the 69th residue from the N-terminus in the amino acid sequence of SEQ ID NO: 5 may be histidine (H, His), but is not limited thereto.

One embodiment of the present disclosure provides a polypeptide, in which an amino acid corresponding to the 69th residue from the N-terminus in the amino acid sequence of SEQ ID NO: 5 is substituted with another amino acid. The polypeptide may be a variant of phosphoribosyl aminoimidazole-succinocarboxamide synthase. The variant of phosphoribosyl aminoimidazole-succinocarboxamide synthase may increase the activity of phosphoribosyl aminoimidazole-succinocarboxamide synthase and/or production ability of purine nucleotides.

In one example, the variant of phosphoribosyl aminoimidazole-succinocarboxamide synthase may be one in which an amino acid corresponding to the 69th amino acid residue of phosphoribosyl aminoimidazole-succinocarboxamide synthase consisting of the amino acid sequence of SEQ ID NO: 5 is substituted with another amino acid selected from the group consisting of, for example, glutamine (Q, Gln), alanine (A, Ala), valine (V, Val), leucine (L, Leu), methionine (M, Met), isoleucine (I, Ile), threonine (T, Thr), asparagine (N, Asn), cysteine (C, Cys), proline (P, Pro), tyrosine (Y, Tyr), tryptophan (W, Trp), lysine (K, Lys), arginine (R, Arg), glycine (G, Gly), aspartic acid (D, Asp), glutamic acid (E, Glu), and serine (S, Ser), and is substituted with an amino acid different from the original amino acid. In one specific embodiment, the variant of phosphoribosyl aminoimidazole-succinocarboxamide synthase may be one in which an amino acid corresponding to the 69th amino acid residue in the amino acid sequence of SEQ ID NO: 5 is substituted with another amino acid, for example, glutamine (Q, Gln), alanine (A, Ala), valine (V, Val), leucine (L, Leu), threonine (T, Thr), asparagine (N, Asn), proline (P, Pro), or serine (S, Ser). It is apparent that, among the variants, even if a portion of the amino acid sequence other than the amino acid corresponding to the 69th amino acid residue in the amino acid sequence of SEQ ID NO: 5 is deleted, modified, substituted, or added, the variant may be included within the scope of the present disclosure as long as it exhibits phosphoribosyl aminoimidazole-succinocarboxamide synthase activity.

In addition, in one embodiment, the variant may comprise a polypeptide in which an amino acid corresponding to the 69th residue of the amino acid sequence of SEQ ID NO: 5 is substituted with another amino acid in an amino acid sequence having at least 80%, 85%, 90%, 91%, 92%, 93%, 94%, 95%, 96%, 97%, 98%, or 99% or more homology or identity with the amino acid sequence of SEQ ID NO: 5. In other words, a polypeptide that comprises a substitution with another amino acid at a position corresponding to the 69th residue of the amino acid sequence of SEQ ID NO: 5, and that comprises/consists of an amino acid sequence having at least 80%, 90%, 95%, 96%, 97%, 98%, or 99% or more and less than 100% sequence homology or identity with the amino acid sequence of SEQ ID NO: 5, and that has phosphoribosyl aminoimidazole-succinocarboxamide synthase activity, may be included in the variant of the present disclosure.

In one specific embodiment, the variant of phosphoribosyl aminoimidazole-succinocarboxamide synthase may comprise an amino acid sequence having 70% or more, 75% or more, 80% or more, 85% or more, 90% or more, 95% or more, 96% or more, 96.3% or more, 96.5% or more, 96.7% or more, 96.9% or more, 97% or more, 97.1% or more, 97.2% or more, 97.4% or more, 97.6% or more, 97.8% or more, 98% or more, 98.2% or more, 98.4% or more, 98.6% or more, 98.9% or more, 99% or more, 99.1% or more, 99.3% or more, 99.5% or more, 99.7% or more, or 99.9% or more homology or identity with the amino acid sequence of any one SEQ ID NO selected from the group consisting of SEQ ID NO: 20 to SEQ ID NO: 27, or consist of the sequence.

Furthermore, a polypeptide having such homology or identity and exhibiting an activity corresponding to that of the phosphoribosyl aminoimidazole-succinocarboxamide synthase variant may be included within the scope of the variant of the present disclosure even if a portion of the sequence is deleted, modified, substituted, conservatively substituted, and/or an amino acid sequence is added. For example, the variant of phosphoribosyl aminoimidazole-succinocarboxamide synthase of the present disclosure may be a case in which additions or deletions of sequences that do not alter the activity of the variant polypeptide, naturally occurring mutations, silent mutations, or conservative substitutions are present at the N-terminus, C-terminus, and/or within the amino acid sequence of the variant polypeptide.

The "conservative substitution"refers to substitution of one amino acid with another amino acid having similar structural and/or chemical properties. Such amino acid substitutions may generally be made based on similarities in residue polarity, charge, solubility, hydrophobicity, hydrophilicity, and/or amphipathic nature. Typically, conservative substitutions have little or no effect on the activity of a protein or polypeptide.

The variant of phosphoribosyl aminoimidazole-succinocarboxamide synthase may have a characteristic to increase production ability of purine nucleotides, as compared with a wild-type polypeptide having the activity of phosphoribosyl aminoimidazole-succinocarboxamide synthase.

In the present disclosure, the term, "purine nucleotides" may be any one nucleotide selected from the group consisting of 5'-inosine monophosphate (hereinafter, IMP), 5'-xanthosine monophosphate (hereinafter, XMP) and 5'-guanosine monophosphate (hereinafter, GMP). The IMP refers to a nucleotide consisting of one molecule each of hypoxanthine, ribose, and phosphate, which is a compound in which adenine is deaminated. IMP may be biosynthesized from 5'-phosphoribosyl1-pyrophosphate (5-phosphoribosyl-1-pyrophosphate; PRPP). Specifically, the pyrophosphate group bound to the C1 carbon of PRPP may be substituted with a nitrogen atom, and through nine steps, an imidazole ring and a pyrimidine ring may be formed to produce IMP. The XMP refers to a nucleotide that is dehydrogenated from IMP. XMP may be synthesized from IMP by inosine-5'-monophosphate dehydrogenase. The GMP refers to a nucleotide having a structure in which a phosphate group forms an ester bond with the ribose moiety of a guanosine molecule. The GMP may be synthesized by addition of an ammonia molecule to XMP by 5'-guanosine monophosphate synthase (GMP synthase). Methods for producing GMP from XMP and/or means used in the methods may be selected from techniques known in the art.

In the present disclosure, the term "variant" refers to a polypeptide that differs from the amino acid sequence prior to mutation due to one or more conservative substitutions and/or modifications, while retaining its functions or properties. Such variants may generally be identified by modifying one or more amino acids in the amino acid sequence of the polypeptide and evaluating the properties of the modified polypeptide. In other words, the activity or capability of a variant may be increased, unchanged, or decreased relative to that of the corresponding pre-mutation polypeptide. In addition, certain variants may include variants in which one or more regions, such as an N-terminal leader sequence or a transmembrane domain, are removed. Other variants may include variants in which a portion of the mature protein is removed from the N-terminus and/or the C-terminus. As used herein, the term "variant" may be used interchangeably with terms such as mutant, modification, modified polypeptide, modified protein, mutation, and variant, and the like (modification, modified polypeptide, modified protein, mutant, mutein, divergent, variant, etc. as Enlgish expressions), and is not limited thereto as long as such terms are used to denote a modified form. For the purposes of the present disclosure, the variant may be a polypeptide in which the amino acid corresponding to the 69th residue of the amino acid sequence of SEQ ID NO: 5 is substituted with another amino acid.

In addition, the variant may comprise deletions or additions of amino acids having minimal impact on the properties and secondary structure of the polypeptide. For example, a signal (or leader) sequence involved in protein translocation co-translationally or post-translationally may be conjugated at the N-terminus of the variant. Furthermore, the variant may be conjugated with another sequence or linker to facilitate identification, purification, or synthesis.

In one embodiment, the variant may comprise any one nucleic acid sequence selected from SEQ ID NO: 29 to SEQ ID NO: 36 or be encoded by a polynucleotide consisting of the nucleic acid sequence.

Another embodiment of the present disclosure provides a polynucleotide encoding the variant of phosphoribosyl aminoimidazole-succinocarboxamide synthase.

In the present disclosure, the term "polynucleotide" refers to a polymer of nucleotides in which nucleotide monomers are covalently linked in a linear chain, which is a DNA or RNA strand having a length of at least a predetermined length.

The polynucleotide encoding the variant of the present disclosure may comprise a nucleotide sequence encoding the amino acid sequence of SEQ ID NO: 5. As one example, the polynucleotide of the present disclosure may comprise a nucleotide sequence set forth in any one selected from SEQ ID NO: 29 to SEQ ID NO: 36, or consist of, or essentially consist of any one SEQ ID NO selected from SEQ ID NO: 29 to SEQ ID NO: 36.

The polynucleotide consisting of any one nucleotide sequence selected from SEQ ID NO: 29 to SEQ ID NO: 36, or comprising the sequence may encode an amino acid sequence set forth in any one selected from SEQ ID NO: 20 to 27, respectively.

The polynucleotide of the present disclosure may be subject to various modifications in its coding region without altering the amino acid sequence of the variant of the present disclosure, in consideration of codon degeneracy and/or codon usage preferred in an organism in which the variant of the present disclosure is to be expressed. Specifically, the polynucleotide of the present disclosure may have or comprise at least 70% or more, 75% or more, 80% or more, 85% or more, 90% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more homology or identity with any one nucleotide sequence selected from SEQ ID NO: 29 to SEQ ID NO: 36, or consist of or essentially consist of a nucleotide sequence having at least 70% or more, 75% or more, 80% or more, 85% or more, 90% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more homology or identity with any one sequence selected from SEQ ID NO: 29 to SEQ ID NO: 36, but is not limited thereto.

The polynucleotide of the present disclosure may include, without limitation, probes that can be prepared from known gene sequences, for example, sequences capable of hybridizing under stringent conditions with a complementary sequence to the whole or a part of the polynucleotide sequence of the present disclosure. The term "stringent conditions" refers to conditions that permit specific hybridization between polynucleotides. Such conditions are described in detail in the literature (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, it may include conditions under which polynucleotides having at least 70% or more, 75% or more, 80% or more, 85% or more, 90% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more homology or identity hybridize with each other, whereas polynucleotides having lower homology or identity do not hybridize, or conditions of washing once, specifically, twice to three times, at the salt concentrations and temperatures corresponding to 60°C, 1×SSC, and 0.1% SDS, more specifically 60°C, 0.1×SSC, and 0.1% SDS, and even more specifically 68°C, 0.1×SSC, and 0.1% SDS, which are washing conditions of conventional southern hybridization.

Hybridization requires that two nucleotides have complementary sequences, however, hybridized polynucleotides may include some base mismatches depending on the stringency of the hybridization. The term "complementary" is used to describe a relationship between nucleotide bases that are capable of hybridizing with each other. For example, with respect to DNA, adenine is complementary to thymine, and cytosine is complementary to guanine. Accordingly, the polynucleotide of the present disclosure may also include substantially similar nucleic acid sequences, as well as isolated nucleic acid fragments complementary to the entire sequence.

Specifically, polynucleotides having homology or identity to the polynucleotide of the present disclosure may be detected using hybridization conditions that include a hybridization step at a Tm value of 55°C and the conditions described above. In addition, the Tm value may be 60°C, 63°C, or 65°C, but is not limited thereto, and may be appropriately adjusted by those skilled in the art depending on the purpose.

The appropriate stringency for hybridizing the polynucleotide depends on the length and degree of complementarity of the polynucleotide, and the variables are well known in the art (for example, J. Sambrook et al., *supra*).

In the present disclosure, that a polynucleotide (which may be used interchangeably with a "gene") or a polypeptide (which may be used interchangeably with a "protein") "comprises a specific nucleic acid sequence or amino acid sequence or consists of or is represented by a specific nucleic acid sequence or amino acid sequence," may mean that the polynucleotide or polypeptide consists of the specific nucleic acid sequence or amino acid sequence, or essentially comprises the same, and it may be interpreted as comprising a "substantially equivalent sequence" (or not excluding the insignificant mutation) in which an insignificant mutation (deletion, substitution, modification, and/or addition) is applied to the specific nucleic acid sequence or amino acid sequence, within a range that maintains the original function and/or an intended function of the polynucleotide or polypeptide.

In the present application, the terms "homology" or "identity" refer to a degree of similarity between two given amino acid sequences or base sequences and may be expressed as a percentage. The terms homology and identity may often be used interchangeably.

The sequence homology or identity of a conserved polynucleotide or polypeptide is determined by a standard alignment algorithm, and default gap penalties established by the program used may be employed together. Substantially, homologous or identical sequences can generally hybridize with the entire sequence or a portion thereof under medium or high stringent conditions. It is apparent that hybridization also includes hybridization with a polynucleotide containing a general codon or codon by considering the codon degeneracy in the polynucleotide.

Whether any two polynucleotide or polypeptide sequences have homology, similarity, or identity may be determined, for example, by using a known computer algorithm such as the "FASTA" program using default parameters as described in Pearson et al. (1988) [Proc. Natl. Acad. Sci. USA 85]: 2444. Otherwise, such determination may be made using the Needleman-Wunsch algorithm (Needleman and Wunsch, 1970, J. Mol. Biol. 48: 443-453), as performed in the Needle program of the EMBOSS package (EMBOSS: The European Molecular Biology Open Software Suite, Rice et al., 2000, Trends Genet. 16: 276-277) (version 5.0.0 or later versions) (including the GCG program package (Devereux, J., et al., Nucleic Acids Research 12: 387 (1984)), BLASTP, BLASTN, FASTA (Altschul, S. F., et al., J. Mol. Biol. 215: 403 (1990); Guide to Huge Computers, Martin J. Bishop, Ed., Academic Press, San Diego, 1994, and Carillo et al. (1988), SIAM J. Applied Math. 48: 1073)). For example, homology, similarity, or identity may be determined using BLAST of the National Center for Biotechnology Information, or ClustalW.

The homology or identity of a polynucleotide or a polypeptide may be determined by comparing sequence information using a GAP computer program such as, for example, Needleman et al. (1970), J Mol Biol. 48:443, as known in, for example, Smith and Waterman, Adv. Appl. Math (1981) 2:482. In summary, the GAP program can be defined as the value obtained by dividing the number of similarly aligned symbols (i.e., nucleotides or amino acids) by the total number of symbols in the shorter of the two sequences. Default parameters for the GAP program may include (1) a binary comparison matrix (containing a value of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix), as disclosed by Schwartz and Dayhoff, eds., Atlas Of Protein Sequence And Structure, National Biomedical Research Foundation, pp. 353-358 (1979); (2) a penalty of 3.0 for each gap and an additional penalty of 0.10 for each symbol in each gap (or a gap opening penalty of 10, a gap extension penalty of 0.5); and (3) no penalty for end gaps.

In the present disclosure, the term "corresponding to" refers to an amino acid residue at a position enumerated in a polypeptide, or an amino acid residue that is similar to, identical to, or homologous to a residue enumerated in the polypeptide. Identifying an amino acid at a corresponding position may be determining a specific amino acid of a sequence with reference to a specific sequence. As used in the present disclosure, the term "corresponding region" generally refers to a similar or corresponding position in a related protein or a reference protein.

For example, an arbitrary amino acid sequence may be aligned with SEQ ID NO: 5, and based thereon, each amino acid residue of the amino acid sequence may be numbered by referring to the numerical position of the amino acid residue corresponding to the amino acid residue of SEQ ID NO: 5. For example, a sequence alignment algorithm as described in the present application may identify positions at which variations such as positions of amino acids, or substitutions, insertions, or deletions occur, as compared with a query sequence (also referred to as a "reference sequence").

For such alignment, for example, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000, Trends Genet. 16: 276-277), etc. can be used, but are not limited thereto, and sequence alignment programs, pairwise sequence comparison algorithms, etc., known in the art can be appropriately used.

Another embodiment of the present disclosure provides a vector comprising a polynucleotide encoding the variant of phosphoribosyl aminoimidazole-succinocarboxamide synthase. The vector may be an insertion vector or expression vector.

In the present disclosure, the term "vector" means a DNA construct for delivering a target polynucleotide into a suitable host or host cell. For example, the vector may comprise a nucleotide sequence of a polynucleotide encoding a target polypeptide operably linked to an appropriate expression regulatory region (or expression regulatory sequence) so as to express the target polypeptide in a suitable host cell, but is not limited thereto. The expression regulatory sequence may comprise a promoter capable of initiating transcription, any operator sequence for regulating transcription, a sequence encoding a suitable mRNA ribosome binding site, and/or a sequence regulating termination of transcription and/or translation. After being transformed into an appropriate host cell, the vector may be maintained independently of the genome (genome) of the host cell, or may be inserted into the genome of the host cell. For example, through an insertion vector, the target polynucleotide may be inserted into a chromosome. The insertion of the polynucleotide into the chromosome may be carried out by any method known in the art, for example, by homologous recombination, but is not limited thereto.

The vector used in the present disclosure is not particularly limited as long as it is capable of replication in a host cell, and may be selected from among all vectors commonly used. Examples of commonly used vectors include plasmids, cosmids, viruses, and bacteriophages, and the like in a natural state or a recombinant state. For example, as the vector, pWE15, M13, MBL3, MBL4, IXII, ASHII, APII, t10, t11, Charon4A, and Charon21A, and the like may be used as phage vectors or cosmid vectors, and pBR-based vectors, pUC-based vectors, pBluescriptII-based vectors, pGEM-based vectors, pTZ-based vectors, pCL-based vectors, and pET-based vectors, and the like may be used as plasmid vectors. Specifically, pDZ, pACYC177, pACYC184, pCL, pECCG117, pUC19, pBR322, pMW118, pCC1BAC and pDC24 vectors, and the like may be exemplified, but is not limited thereto.

The vector may further comprise a selection marker for confirming whether the vector is introduced into transformed cells or whether the vector is inserted into the genome of the transformed cells. The selection marker is for selecting cells transformed with the vector, or insertion of the polynucleotide, and may be selected and used from genes that confer a selectable phenotype such as drug resistance, auxotrophy, resistance to cytotoxic agents, or expression of a surface protein. In an environment treated with a selective agent, only cells expressing the selection marker survive or exhibit other phenotypic traits, thereby allowing selection of transformed cells.

The expression of the variant in a microorganism may be performed by introducing a polynucleotide encoding the variant, or a vector comprising the same, into a host cell and culturing a recombinant cell (e.g., a microorganism) comprising the same.

The introduction of the polynucleotide encoding the variant or the vector comprising the same into a microorganism may be performed by appropriately selecting a known transformation method by those skilled in the art. In the present disclosure, the term "transformation" means introducing a target polynucleotide or a vector comprising the same into a host cell (microorganism) to change the genetic traits of the host cell (microorganism). The transformed polynucleotide may be inserted into the chromosome of the host cell or may be located extrachromosomally. The polynucleotide may be introduced in an appropriate form depending on the purpose of introduction. For example, the polynucleotide may be introduced into a host cell in the form of an expression cassette, which is a gene construct comprising all elements necessary for autonomous expression. The expression cassette may typically comprise expression regulatory elements such as a promoter operably linked to the polynucleotide, a transcription termination signal, a ribosome binding site, and/or a translation termination signal, and the like. The expression cassette may be in the form of an expression vector capable of autonomous replication. In addition, the polynucleotide may be introduced into a host cell in its own form and operably linked to sequences required for expression in the host cell. Herein, the term "operably linked" may mean that an expression regulatory element (e.g., a promoter) and the polynucleotide are functionally linked so as to perform transcriptional regulation (e.g., initiation of transcription) of the polynucleotide. Operable linkage may be carried out using known gene recombination techniques in the art.

A method for transforming the polynucleotide into a host microorganism may be carried out by any method of introducing a nucleic acid into a cell (microorganism), and may be performed by appropriately selecting, depending on the host microorganism, a transformation technique known in the art. Examples of the known transformation methods may include electroporation, calcium phosphate (CaPO₄) precipitation, calcium chloride (CaCl₂) precipitation, microinjection, polyethylene glycol (PEG) precipitation (polyethylene glycol-mediated uptake), DEAE-dextran method, cationic liposome method, lipofection, and lithium acetate-DMSO method, and the like, but are not limited thereto.

Other embodiment of the present disclosure provides a microorganism comprising the phosphoribosyl aminoimidazole-succinocarboxamide synthase variant.

Specifically, the microorganism may be a microorganism comprising at least one (for example, at least one, at least two, or one kind, 2 kinds, or 3 kinds) selected from the group consisting of the polypeptide (variant) having phosphoribosyl aminoimidazole-succinocarboxamide synthase activity described above, a polynucleotide encoding (or coding) the polypeptide, and a vector comprising the polynucleotide.

In the present disclosure, the term, "microorganism (or, strain)" may include both a wild-type microorganism, or a microorganism in which genetic modification occurs naturally or artificially. The microorganism may be a microorganism in which a specific mechanism is enhanced or weakened due to insertion of an exogenous gene or enhancement or attenuation of the activity of an endogenous gene, and may be a microorganism comprising a genetic modification for the production of a desired polypeptide, protein, or product (for example, purine nucleotides). In the present disclosure, the terms "microorganism," "strain," "host," and "host cell" may be used interchangeably.

The microorganism (or strain, recombinant cell) of the present disclosure may be a microorganism in which the activity of phosphoribosyl aminoimidazole-succinocarboxamide synthase is enhanced, or which has a production ability (production amount) of purine nucleotides, or which has an enhanced (or increased) ability to produce purine nucleotides.

As one example, the microorganism of the present disclosure may be a microorganism that naturally does not have purine nucleotide production ability, or a microorganism into which purine nucleotide production ability is imparted or enhanced by introducing the polypeptide (variant) having phosphoribosyl aminoimidazole-succinocarboxamide synthase activity of the present disclosure or a polynucleotide encoding the same into a microorganism having purine nucleotide production ability, but is not limited thereto.

In the present disclosure, the term "microorganism comprising a phosphoribosyl aminoimidazole-succinocarboxamide synthase variant" may refer to a microorganism that has enhanced phosphoribosyl aminoimidazole-succinocarboxamide synthase activity, or a microorganism that previously did not have the purine nucleotide production ability and acquires the purine nucleotide production ability, or one having purine nucleotide production ability higher than the original purine nucleotide production ability, by being manipulated (modified) to express the polypeptide (variant) having phosphoribosyl aminoimidazole-succinocarboxamide synthase activity described above.

In the present disclosure, "non-modified microorganism" does not exclude strains that include mutations that may occur naturally in microorganisms, and may refer to a wild-type strain or a native strain itself, or a strain prior to alteration of its phenotypes due to genetic variation caused by natural or artificial factors. For example, the non-modified microorganism may, according to one embodiment, refer to a strain into which the polypeptide (variant) having phosphoribosyl aminoimidazole-succinocarboxamide synthase activity of the present disclosure or a polynucleotide encoding the polypeptide (variant) having phosphoribosyl aminoimidazole-succinocarboxamide synthase activity has not been introduced, or a strain prior to such introduction. The term "non-modified microorganism" may be used interchangeably with "strain before modification," "microorganism before modification," "non-mutant strain," "non-modified microorganism," "non-mutant microorganism," or "reference microorganism."

In the present disclosure, the reference microorganism may be a wild-type microorganism known to produce purine nucleotides, and for example, may be *Corynebacterium stationis* ATCC6872. Otherwise, the reference microorganism may be a microorganism known to produce purine nucleotides, and for example, may be *Corynebacterium stationis* KCCM12151P (US 2023-0192780 A1) or *Corynebacterium stationis* CJX1664 (KCCM12285P, Korean Patent No. 10-1950141, but is not limited thereto.

The microorganism producing purine nucleotides of the present disclosure is not particularly limited, as long as purine nucleotides can be contemplated, but may be a microorganism of the genus *Corynebacterium*. The microorganism of the genus *Corynebacterium* may be at least one microorganism selected from the group consisting of *Corynebacterium stationis*, *Corynebacterium thermoaminogenes*, *Corynebacterium glutamicum*, *Brevibacterium flavum*, *Brevibacterium lactofermentum*, *Corynebacterium crudilactis*, *Corynebacterium deserti*, *Corynebacterium efficiens*, *Corynebacterium callunae*, *Corynebacterium singulare*, *Corynebacterium halotolerans*, *Corynebacterium striatum*, *Corynebacterium pollutisoli*, *Corynebacterium imitans*, *Corynebacterium testudinoris*, and *Corynebacterium flavescens*, but is not limited thereto. Specifically, the microorganism of the genus *Corynebacterium* may be *Corynebacterium stationis*.

Other embodiment of the present disclosure provides a method for producing purine nucleotides, comprising culturing a microorganism with enhanced activity of phosphoribosyl aminoimidazole-succinocarboxamide synthase in a medium.

The microorganism may be a microorganism comprising at least one (for example, at least one, at least two, or one kind, 2 kinds, or 3 kinds) selected from the group consisting of the polypeptide (variant) having activity of phosphoribosyl aminoimidazole-succinocarboxamide synthase described above, a polynucleotide encoding (or coding) the polypeptide, and a vector comprising the polynucleotide.

The phosphoribosyl aminoimidazole-succinocarboxamide synthase, variant, microorganism, and purine nucleotides are as described above.

In the present disclosure, "culturing" means growing a microorganism into which the polypeptide having activity of phosphoribosyl aminoimidazole-succinocarboxamide synthase of the present disclosure or a gene encoding the same is introduced, or in which activity thereof is enhanced, for example, a microorganism of the genus *Corynebacterium*, under appropriately controlled environmental conditions. The culturing process of the present disclosure may be performed according to suitable media and culturing conditions known in the art. Such a culturing process may be readily adjusted and used by those skilled in the art depending on the strain selected. Specifically, the culturing may be a batch manner, a continuous manner, and/or a fed-batch manner, but is not limited thereto.

In the present disclosure, "medium" means a substance mainly comprising nutrients required for culturing a microorganism into which the polypeptide having activity of phosphoribosylaminoimidazole-succinocarboxamide synthase of the present disclosure or a gene encoding the same is introduced, or in which activity thereof is enhanced, for example, a microorganism of the genus *Corynebacterium*, and supplies water indispensable for survival and growth, as well as nutrients and growth factors. In addition, the medium may further comprise XMP for GMP synthesis. Specifically, the medium used for culturing the microorganism of the present disclosure and other culturing conditions may be any medium commonly used for culturing microorganisms without particular limitation, but the microorganism of the present disclosure may be cultured under aerobic conditions while controlling temperature, pH, and the like in a conventional medium containing appropriate carbon sources, nitrogen sources, phosphorus sources, inorganic compounds, amino acids, and/or vitamins, and the like.

In the present disclosure, the carbon source may include carbohydrates such as glucose, saccharose, lactose, fructose, sucrose, maltose, etc.; sugar alcohols such as mannitol, sorbitol, etc.; organic acids such as pyruvic acid, lactic acid, citric acid, etc.; and amino acids such as glutamic acid, methionine, lysine, etc., and the like. In addition, natural organic nutrient sources such as starch hydrolysates, molasses (for example, blackstrap molasses), rice bran, cassava, sugarcane bagasse, and corn steep liquor may be used, and specifically, carbohydrates such as glucose and sterilized pretreated molasses (that is, molasses converted into reducing sugars) may be used, and other carbon sources in appropriate amounts may be variously used without limitation. These carbon sources may be used alone or in combination of two or more, and are not limited thereto.

As the nitrogen source, inorganic nitrogen sources such as ammonia, ammonium sulfate, ammonium chloride, ammonium acetate, ammonium phosphate, ammonium carbonate, ammonium nitrate, etc.; amino acids such as glutamic acid, methionine, glutamine, etc.; and organic nitrogen sources such as peptone, NZ-amine, meat extract, yeast extract, malt extract, corn steep liquor, casein hydrolysate, fish or degradation products thereof, and defatted soybean cake or degradation products thereof may be used. These nitrogen sources may be used alone or in combination of two or more, without limitation.

As the phosphorus source, potassium dihydrogen phosphate, dipotassium hydrogen phosphate, or corresponding sodium-containing salts may be included. As inorganic compounds, sodium chloride, calcium chloride, ferric chloride, magnesium sulfate, ferric sulfate, manganese sulfate, calcium carbonate, and the like may be used, and in addition thereto, amino acids, vitamins, and/or appropriate precursors, and the like may be included. These components or precursors may be added to the medium in a batch or continuous manner. However, it is not limited thereto.

In addition, during the culturing of the microorganism of the present disclosure, the pH of the medium may be adjusted by adding compounds such as ammonium hydroxide, potassium hydroxide, ammonia, phosphoric acid, sulfuric acid, and the like, to the medium in an appropriate manner. In addition, during the culturing, bubble generation may be suppressed by using an antifoaming agent such as fatty acid polyglycol ester. Further, in order to maintain an aerobic condition of the medium, oxygen or an oxygen-containing gas may be injected into the medium, or in order to maintain anaerobic or microaerobic conditions, the medium may be maintained without gas injection or by injecting nitrogen, hydrogen, or carbon dioxide gas, but is not limited thereto.

In the culturing of the present disclosure, the culturing temperature may be maintained at 20 to 45°C, or 25 to 37°C, specifically, 25 to 37°C, and the culturing may be performed for about 10 to 160 hours, or about 20 to 120 hours, but are not limited thereto.

The purine nucleotides produced by the culturing of the present disclosure may be secreted inot the medium or remain within cells.

The method for producing purine nucleotides of the present disclosure, may comprise adding an enzyme to the medium or adding a microorganism expressing the enzyme. For example, the method may further comprise adding an enzyme that converts XMP into GMP or a microorganism expressing the enzyme, and/or culturing the microorganism, after the culturing a microorganism that produces XMP.

In one embodiment, the method for producing purine nucleotides of the present disclosure may further comprise culturing a microorganism producing 5'-xanthosine monophosphate (XMP) or adding XMP to the medium, before the culturing a microorganism with enhanced activity of phosphoribosyl aminoimidazole-succinocarboxamide synthase in a medium.

The method for producing purine nucleotides of the present disclosure, may further comprise recovering purine nucleotides from the cultured microorganism (for example, a microorganism of the genus *Corynebacterium*), a medium resulting from the culturing (medium in which the culturing is performed) or both of them. The recovering may be further comprised after the culturing.

The recovering may be collecting the desired purine nucleotides using an appropriate method known in the art, depending on the culturing method, for example, a batch, continuous, or fed-batch culturing method, of the microorganism of the present disclosure. For example, centrifugation, filtration, crystallization, treatment with a protein precipitating agent (salting-out), extraction, ultrasonic disruption, ultrafiltration, dialysis, various types of chromatography such as molecular sieve chromatography (gel filtration), adsorption chromatography, ionexchange chromatography, affinity chromatography, HPLC, or combinations of these methods may be used, and the desired purine nucleotides may be recovered from the medium or microorganism using an appropriate method known in the art.

In addition, the method for producing purine nucleotides of the present disclosure may further comprise a purifying. The purifying may be performed using an appropriate method known in the art. In one embodiment, when the method for producing purine nucleotides of the present disclosure comprises both the recovering and the purifying, the recovering and the purifying may be performed sequentially or non-sequentially regardless of order, or may be performed simultaneously or integrated into a single step, without limitation.

Other embodiment of the present disclosure provides a composition for producing purine nucleotides comprising a microorganism with enhanced activity of phosphoribosyl aminoimidazole-succinocarboxamide synthase, a medium in which the microorganism is cultured, or a combination thereof.

The microorganism with enhanced activity of phosphoribosyl aminoimidazole-succinocarboxamide synthase, medium, and purine nucleotides are as described above.

Other embodiment provides a use of the microorganism for producing purine nucleotides.

Other embodiment provides a use of the microorganism for the preparation of a composition for producing purine nucleotides.

The composition of the present disclosure may further comprise any appropriate excipient commonly used in the composition for producing purine nucleotides, and such an excipient, may be for example, a preservative, a wetting agent, a dispersant, a suspending agent, a buffer, a stabilizer, or an emulsifying agent, but is not limited thereto.

### [ADVANTAGEOUS EFFECTS]

The present disclosure relates to a novel phosphoribosyl aminoimidazole-succinocarboxamide synthase variant, a microorganism comprising the phosphoribosyl aminoimidazole-succinocarboxamide synthase variant, a composition for producing purine nucleotides comprising the microorganism, and a method for producing purine nucleotides comprising culturing the microorganism, and enables high-yield production of purine nucleotides by culturing a microorganism of the genus *Corynebacterium* into which the phosphoribosyl aminoimidazole-succinocarboxamide synthase variant of the present disclosure is introduced.

### [MODE FOR INVENTION]

Hereinafter, the present disclosure will be described in more detail by examples. However, the following examples are merely preferred embodiments for illustrating the present disclosure and accordingly, are not intended to limit the scope of the present disclosure thereto. Meanwhile, technical matters not described in the present disclosure can be sufficiently understood and easily conducted by those skilled in the art in the technical field of the present disclosure or similar technical fields.

### Example 1: Identification of phosphoribosylaminoimidazole-succinocarboxamide synthase gene mutations

A mutation library of the *purC* gene encoding phosphoribosylaminoimidazole-succinocarboxamide synthase, which is one of the IMP biosynthetic enzymes, was constructed, and enhancing mutations that increase IMP production ability were intended to be identified.

### Example 1-1: Construction of a vector comprising purC

In order to construct a purC library, a recombinant vector comprising the *purC* gene was constructed as follows using plasmid pDC24 (SEQ ID NO: 37) for insertion and replacement of genes in the *Corynebacterium* chromosome.

Specifically, chromosomal genes of a wild-type *Corynebacterium stationis* ATCC 6872 strain were isolated using a G-spin Total DNA extraction mini kit (Cat. No. 17045) from Intron company according to the protocol provided in the kit, and a *purC* gene fragment was obtained by performing a polymerase chain reaction using a primer pair of SEQ ID NO: 1 and SEQ ID NO: 2. The PCR conditions were after denaturation at 94°C for 5 minutes, repeating denaturation at 94°C for 30 seconds, annealing at 55°C for 30 seconds, and polymerization at 72°C for 2 minutes 20 times, followed by a polymerization reaction at 72°C for 7 minutes.

The gene fragment obtained above was cloned into linear pDC24 cut with a SmaI restriction enzyme through a Gibson assembly (NEB) method to obtain pDC24-*purC*. The Gibson assembly reaction (based on 20ul) was carried out by mixing 1 ul of linear pDC24 vector, 3 ul of *purC* PCR DNA, 10 ul of Gibson assembly master mix, and 3 ul of PCR grade water, and reacting at 50°C for 30 minutes.

### 1-2: Construction of a purC mutagenesis library

Based on the vector constructed in Example 1-1 above, a purC mutagenesis library was constructed by the following method.

Specifically, in order to introduce random mutations into the *purC* gene, an Error-Prone PCR technique was used. The pDC24-*purC* constructed in Example 1-1 above was used as a PCR template, and a reaction was carried out using a primer pair of SEQ ID NO: 1 and SEQ ID NO: 2, and PCR buffer conditions were set with the objective of introducing mutations of 2.0 bp per 1 kb, and the reaction was carried out with reference to the manufacturer's manual (Diversify PCR Random Mutagenesis Kit, TAKARA). The Error-Prone PCR reaction (based on 50 µl) was carried out with a composition of 40 ul of PCR grade water, 5 µl of 10X TITANIUM Taq buffer, 1 µl of 2 mM dGTP, 1 µl of 50X Diversify dNTP Mix, 1 µl of primer mix, 1 µl of template DNA, and 1 µl of TITANIUM Taq polymerase, and the PCR conditions were after denaturation at 94°C for 30 seconds, repeating denaturation at 94°C for 30 seconds and polymerization at 68°C for 2 minutes 25 times, followed by a polymerization reaction at 68°C for 1 minute. A *purC* gene fragment, which was expected to have random mutations introduced therein, was cloned into pDC24 to obtain pDC24-*purC*(Mut).

### 1-3: Construction of a Corynebacterium stationis strain library comprising a purC variant vector library

Using the pDC24-*purC*(Mut) variant vector library constructed in Example 1-2, an IMP-producing strain, *Corynebacterium stationis* KCCM12151P (US 2023-0192780 A1), was transformed by electroporation, and then spread on a selective medium containing 25 mg/L kanamycin, thereby securing 10,000 colonies of strains into which mutant genes were inserted and selecting them as a primary candidate group. The selected strain library was named KCCM12151P_purC(library_1)~ KCCM12151P_purC(library_10000), respectively.

In addition, for use as a control in the experiment, a strain was prepared by introducing the pDC24-purC vector into *Corynebacterium stationis* KCCM12151P in the same manner, and the strain was named KCCM12151P_purC(WT).

### 1-4: Evaluation of the constructed purC library and selection of strains

Each of the 10,000 colonies obtained in Example 1-3 above was inoculated into 200 µl of autoclave-sterilized seed medium, and cultured by shaking in a 96 deep well plate using a microplate shaker (TAITEC) at 30°C and 1200 rpm for 24 hours, thereby using as a seed culture. After dispensing 290 µl of autoclave-sterilized fermentation medium into a 96 deep well plate, 20 µℓ of the seed culture was inoculated into each well, and shaking culture was performed for 72 hours under the same conditions as described above.

In order to analyze the production amount of 5'-inosine monophosphate produced in the culture broth, after completion of the culture, 3 µl of the culture supernatant was transferred to a 96-well UV plate into which 197 µl of distilled water had been dispensed. Next, using a microplate reader, shaking was performed for 30 seconds, and absorbance was measured with a spectrophotometer at 25°C and a wavelength of 270 nm, and 50 colonies of mutant strains showing an absorbance increased by 10% or more as compared with the absorbance of the KCCM12151P_purC(WT) strain were selected. The other colonies showed similar or decreased absorbance as compared with the control.

The selected 50 strains were repeatedly subjected to confirmation of 5'-inosine monophosphate production by absorbance measurement in the same manner as described above, and one strain, KCCM12151P_purC(library_708), showing a level of 5'-inosine monophosphate production comparable to that of the KCCM12151P_purC(WT) strain, and one strain, KCCM12151P_purC(library_3291), showing a significantly improved 5'-inosine monophosphate production ability, were selected.

### Example 1-5: Confirmation of purC mutations by gene sequencing

In order to confirm gene mutations of two mutant strains selected in Example 1-4 above, PCR was performed, and sequencing was carried out, on the strains KCCM12151P_purC(library_708) and KCCM12151P_purC(library_3291) using a primer pair of SEQ ID NO: 3 and SEQ ID NO: 4, followed by comparison with the wild-type purC gene sequence of the *Corynebacterium stationis* KCCM12151P strain.

As a result, it was confirmed that both strains included one amino acid mutation each in the purC gene. Specifically, it was confirmed that the KCCM12151P_purC(library_708) strain comprised a mutation in which the 29th methionine was substituted with leucine in the amino acid sequence encoded by the purC gene represented by SEQ ID NO: 5, and that the KCCM12151P_purC(library_3291) strain comprised a mutation in which the 69th histidine was substituted with glutamine in the amino acid sequence encoded by the purC gene represented by SEQ ID NO: 5.

The primer sequences used in Example 1 are shown in Table 1 below.

**[Table 1]**

| Name | Sequence (5'->3') | SEQ ID NO: |
|---|---|---|
| Primer 1 | TTCGAGCTCGGTACCCGTCAGCAGTGGAACGAA GGCGAC | SEQ ID NO: 1 |
| Primer 2 | CTCTAGAGGATccccAGATATCGGTCAGGTGGTC ATCG | SEQ ID NO: 2 |
| Primer 3 | GGTAAGAGTCCAGAAGAA | SEQ ID NO: 3 |
| Primer 4 | CGCATCCACTCATATTCA | SEQ ID NO: 4 |

### Example 2: Construction of strains into which purC mutations were introduced and evaluation of 5'-inosine monophosphate production ability

### Example 2-1: Construction of recombinant vectors for introducing purC mutations

In order to confirm the effects of the M29L mutation and the H69Q mutation in the amino acid sequence encoded by the purC gene identified in Example 1-5 above on IMP production ability, vectors for introducing the mutations into the endogenous purC gene of a *Corynebacterium stationis* strain were constructed.

Specifically, plasmid pDC24 (SEQ ID NO: 37) for insertion and replacement of genes in the chromosome of a *Corynebacterium stationis* strain was used to construct the vectors as follows.

Using the genomes of the KCCM12151P_purC(library_708) strain and the KCCM12151P_purC(library_3291) strain selected in Example 1-4 above as templates, gene fragments purC-M29L and purC-H69Q were obtained, respectively, by performing a polymerase chain reaction using a primer pair of SEQ ID NO: 1 and SEQ ID NO: 2. The PCR conditions were after denaturation at 94°C for 5 minutes, repeating denaturation at 94°C for 30 seconds, annealing at 55°C for 30 seconds, and polymerization at 72°C for 2 minutes 20 times, followed by a polymerization reaction at 72°C for 7 minutes. The gene fragments obtained above were cloned into linear pDC24 cut with a SmaI restriction enzyme through a Gibson assembly (NEB) method to obtain pDC24-purC(M29L) and pDC24-purC(H69Q), respectively.

### Example 2-2: Construction of 5'-inosine monophosphate-producing strains into which purC mutations were introduced

In order to confirm the effects of the M29L mutation and the H69Q mutation in the amino acid sequence encoded by the purC gene identified in Example 1-5 above on IMP production ability, strains into which the mutations were introduced into the endogenous purC gene of a *Corynebacterium stationis* strain were constructed.

The pDC24-purC(M29L) vector and the pDC24-purC(H69Q) vector constructed in Example 2-1 above were each transformed by electroporation into *Corynebacterium stationis* KCCM12151P, which is a strain having 5'-inosine monophosphate production ability, and strains in which the mutant gene and the vector were inserted together into the chromosome were selected as a primary candidate group on a selective medium containing 25 mg/L kanamycin. Thereafter, in strains in which homologous recombination had occurred, secondary confirmation was performed by PCR using a primer pair of SEQ ID NO: 3 and SEQ ID NO: 4, followed by final confirmation through gene sequence analysis. The selected strains were named CJI-3414 (KCCM12151P_purC(M29L)) and CJI-3415 (KCCM12151P_purC(H69Q)), respectively.

### Example 2-3: Evaluation of 5'-inosine monophosphate production ability of 5'-inosine monophosphate-producing strains into which purC mutations were introduced

In order to measure the 5'-inosine monophosphate production ability of the CJI-3414 and CJI-3415 strains constructed in Example 2-2 above, a flask titer evaluation was performed.

Specifically, *Corynebacterium stationis* KCCM12151P, CJI-3414, and CJI-3415 strains were each inoculated into a 14 mL tube containing 2.5 mL of the seed medium described below, and shaking culture was performed at 30°C and 170 rpm for 24 hours. Then, 2 mL of the seed culture was inoculated into a 250 mL corner-baffled flask containing 29 mL of the production medium described below (24 mL of main medium + 5 mL of separately sterilized medium), and shaking culture was performed at 30°C and 170 rpm for 72 hours. After completion of the culture, OD (Optical Density) was measured at a wavelength of 562 nm using a spectrophotometer (Eppendorf). In addition, the production amount of 5'-inosine monophosphate was measured using HPLC (UC11-SHIMADZU), and specifically, inosine-5-monophosphate disodium salt hydrate (aldrich, 57510-5G) was used as a standard reagent, and as a mobile phase for analysis, 10 g of 0.2% ammonium dihydrogen phosphate, 1 g of 0.02% tetrabutylammonium phosphate monobasic, and 108 mL of 2.1% acetonitrile were added to 5 L of distilled water, and the pH was adjusted to 2.4 with H₃PO₄. The column temperature was 40°C, the flow rate was 1.0 mL/min, and a culture-completed sample diluted 20-fold was injected in an amount of 2 µL for measurement. The culture results are shown in Table 2 below.

### <Composition of seed medium for IMP production>

Glucose 1%, peptone 1%, meat extract 1%, yeast extract 1%, sodium chloride 0.25%, adenine 100 mg/L, guanine 100 mg/L, pH 7.2

### <Composition of production medium for IMP production>

Sodium glutamate 0.1%, ammonium chloride 1%, magnesium sulfate 1.2%, calcium chloride 0.01%, ferrrous sulfate 20 mg/L, manganese sulfate 20 mg/L, zinc sulfate 20 mg/L, copper sulfate 5 mg/L, L-cysteine 23 mg/L, beta-alanine 24 mg/L, nicotic acid 8 mg/L, biotin 45 µg/L, thiamine hydrochloride 5 mg/L, adenine 30 mg/L, phosphoric acid(85%) 1.9%, glucose 4.2%, fructose 2.4%

**[Table 2]**

| Confirmation of 5'-inosine monophosphate production amount according to introduction of purC gene mutation | | | | |
|---|---|---|---|---|
| Strain name | Form introduced | OD | 5'-inosine monophosphate(g/L) | Concentration increase rate (%) |
| KCCM12151P | Control | 39.1 | 4.9 | - |
| CJI-3414 | purC(M29L) | 39.2 | 5 | 2 |
| CJI-3415 | purC(H69Q) | 39.6 | 5.4 | 10.2 |

As a result, as shown in Table 2, the CJI-3414 strain, into which the purC(M29L) mutation was introduced into an IMP-producing strain, exhibited a result similar to that of the control, with the concentration of 5'-inosine monophosphate increased by about 2% as compared with the KCCM12151P strain, whereas the CJI-3415 strain, into which the purC(H69Q) mutation was introduced, was confirmed to exhibit an increase of about 10.2% in the concentration of 5'-inosine monophosphate as compared with the KCCM12151P strain.

### Example 3: Construction of strains into which mutations in which the amino acid at position 69 in the amino acid sequence encoded by the purC gene is substituted with an amino acid other than glutamine are introduced and evaluation of 5'-inosine monophosphate production ability

### Example 3-1: Construction of vectors for insertion of purC(H67) mutation amino acid substitutions

Through Example 2 above, it was confirmed that the H69Q mutation in the amino acid sequence encoded by the purC gene could improve inosine monophosphate production ability. Accordingly, in order to confirm the positional importance of PurC(H67), vectors for substituting the 69th amino acid with an amino acid other than glutamine were constructed, and it was confirmed whether the substitution affects 5'-inosine monophosphate production ability.

Specifically, site-directed mutagenesis was performed using the pDC24-purC(H69Q) vector constructed in Example 2-1 above as a template. Site-directed PCR was performed using primer pairs of SEQ ID NO: 1 and SEQ ID NO: 6 and SEQ ID NO: 2 and SEQ ID NO: 7 for introduction of a purC(H69A) mutation; primer pairs of SEQ ID NO: 1 and SEQ ID NO: 8 and SEQ ID NO: 2 and SEQ ID NO: 9 for introduction of a purC(H69V) mutation; primer pairs of SEQ ID NO: 1 and SEQ ID NO: 10 and SEQ ID NO: 2 and SEQ ID NO: 11 for introduction of a purC(H69L) mutation; primer pairs of SEQ ID NO: 1 and SEQ ID NO: 12 and SEQ ID NO: 2 and SEQ ID NO: 13 for introduction of a purC(H69T) mutation; primer pairs of SEQ ID NO: 1 and SEQ ID NO: 14 and SEQ ID NO: 2 and SEQ ID NO: 15 for introduction of a purC(H69N) mutation; primer pairs of SEQ ID NO: 1 and SEQ ID NO: 16 and SEQ ID NO: 2 and SEQ ID NO: 17 for introduction of a purC(H69P) mutation; and primer pairs of SEQ ID NO: 1 and SEQ ID NO: 18 and SEQ ID NO: 2 and SEQ ID NO: 19 for introduction of a purC(H69S) mutation, respectively, and at this time, after denaturation at 94°C for 5 minutes, denaturation at 94°C for 30 seconds, annealing at 55°C for 30 seconds, and polymerization at 72°C for 2 minutes were repeated 20 times, followed by a polymerization reaction at 72°C for 7 minutes. As a result, each PCR product was obtained, and after DpnI treatment, the PCR products were cloned into linearized pDC24 cut with a SmaI restriction enzyme through a Gibson assembly (NEB) method to obtain plasmids in which the 69th amino acid of purC was modified to the targeted amino acid, and information on the obtained plasmids is shown in Table 3 below.

**[Table 3]**

| List of vectors for insertion of purC(H69) mutation amino acid substitution | |
|---|---|
| No. | Plasmid name |
| 1 | pDC24-purC(H69A) |
| 2 | pDC24-purC(H69V) |
| 3 | pDC24-purC(H69L) |
| 4 | pDC24-purC(H69T) |
| 5 | pDC24-purC(H69N) |
| 6 | pDC24-purC(H69P) |
| 7 | pDC24-purC(H69S) |

The primer sequences used in Example 3-1 are shown in Table 1 and Table 4 below.

**[Table 4]**

| Name | Sequence (5' → 3') |
|---|---|
| SEQ ID NO: 6 | CGATGGGTCCTGCCAAagcGTTCGGGAAATCGATGGC |
| SEQ ID NO: 7 | TCGATTTCCCGAACgctTTGGCAGGACCCATCGATG |
| SEQ ID NO: 8 | CGATGGGTCCTGCCAAcacGTTCGGGAAATCGATGGC |
| SEQ ID NO: 9 | TCGATTTCCCGAACgtgTTGGCAGGACCCATCGATG |
| SEQ ID NO: 10 | CGATGGGTCCTGCCAAgagGTTCGGGAAATCGATGGC |
| SEQ ID NO: 11 | TCGATTTCCCGAACctcTTGGCAGGACCCATCGATG |
| SEQ ID NO: 12 | CGATGGGTCCTGCCAAggtGTTCGGGAAATCGATGGC |
| SEQ ID NO: 13 | TCGATTTCCCGAACaccTTGGCAGGACCCATCGATG |
| SEQ ID NO: 14 | CGATGGGTCCTGCCAAgttGTTCGGGAAATCGATGGC |
| SEQ ID NO: 15 | TCGATTTCCCGAACaacTTGGCAGGACCCATCGATG |
| SEQ ID NO: 16 | CGATGGGTCCTGCCAAtggGTTCGGGAAATCGATGGC |
| SEQ ID NO: 17 | TCGATTTCCCGAACccaTTGGCAGGACCCATCGATG |
| SEQ ID NO: 18 | CGATGGGTCCTGCCAAcgaGTTCGGGAAATCGATGGC |
| SEQ ID NO: 19 | TCGATTTCCCGAACtcgTTGGCAGGACCCATCGATG |

### Example 3-2: Construction of strains into which mutations in which the amino acid at position 69 in PurC is substituted with an amino acid other than glutamine are introduced

The seven kinds of vectors constructed in Example 3-1 above were each transformed by electroporation into *Corynebacterium stationis* KCCM12151P, which is a strain having 5'-inosine monophosphate production ability, and strains in which the mutant gene and the vector were inserted together into the chromosome on a selective medium containing 25 mg/L kanamycin were selected as a primary candidate group. Thereafter, in strains in which homologous recombination had occurred, secondary confirmation was performed by PCR using a primer pair of SEQ ID NO: 3 and SEQ ID NO: 4, followed by final confirmation through gene sequence analysis. The names of the strains according to the inserted mutations are shown in Table 5 below.

**[Table 5]**

| No. | Strain name |
|---|---|
| 1 | KCCM12151P::purC(H69A) |
| 2 | KCCM12151P::purC(H69V) |
| 3 | KCCM12151P::purC(H69L) |
| 4 | KCCM12151P::purC(H69T) |
| 5 | KCCM12151P::purC(H69N) |
| 6 | KCCM12151P::purC(H69P) |
| 7 | KCCM12151P::purC(H69S) |

### Example 3-3. Confirmation of 5'-inosine monophosphate production ability of strains into which mutations in which the amino acid at position 69 in PurC is substituted with an amino acid other than glutamine are introduced

In order to measure the 5'-inosine monophosphate production ability of the seven kinds of strains constructed in Example 3-2 above, a flask titer evaluation was performed in the same manner as the method of Example 2-3 to measure the production amount of 5'-inosine monophosphate, and the culture results of strains, into which mutations in which the amino acid at position 69 in PurC was substituted with an amino acid other than glutamine were introduced into *Corynebacterium stationis* KCCM12151P, which is an IMP-producing strain, are shown in Table 6 below.

**[Table 6]**

| Strain No. | Form introduced | OD | 5'-inosine monophosphate(g/L) | Concentration increase rate (%) |
|---|---|---|---|---|
| KCCM12151P | Control | 39.1 | 4.9 | - |
| CJI-3415 | purC(H69Q) | 39.7 | 5.5 | 12.2 |
| 1 | purC(H69A) | 39.2 | 5.1 | 4.1 |
| 2 | purC(H69V) | 39.1 | 5.2 | 6.1 |
| 3 | purC(H69L) | 39.4 | 5.1 | 4.1 |
| 4 | purC(H69T) | 39.8 | 5.3 | 8.2 |
| 5 | purC(H69N) | 39.8 | 5.3 | 8.2 |
| 6 | purC(H69P) | 39.5 | 5.1 | 4.1 |
| 7 | purC(H69S) | 39.6 | 5.2 | 6.1 |

As a result, as shown in Table 6, it could be confirmed that strains comprising the purC mutant gene in which the 69th amino acid in the amino acid sequence encoded by the purC gene was substituted with another amino acid exhibited an increased IMP production amount as compared with the KCCM12151P strain that does not include the mutation. In other words, it was confirmed that the 69th amino acid in the amino acid sequence encoded by the purC gene is a major mutation position in inosine monophosphate production. More specifically, it was confirmed that the 5'-inosine monophosphate production amount of microorganisms including mutations in which the 69th amino acid in the amino acid sequence encoded by the purC gene was substituted with glutamine, alanine, valine, leucine, threonine, asparagine, proline, or serine was significantly increased.

### Example 4: Construction of strains into which purC mutations were introduced and evaluation of 5'-xanthosine monophosphate production ability

### Example 4-1: Construction of 5'-xanthosine monophosphate-producing strains into which purC mutations were introduced

In order to confirm whether a mutation of the purC gene can lead to an increase in the production amount of XMP, a purC(H69Q) mutation was introduced into the purC gene of *Corynebacterium stationis* CJX1664 (US 11697810 B2), which is an XMP-producing strain, in the same manner as described below.

Specifically, the pDC24-purC(H69Q) vector constructed in Example 2-1 above was transformed by electroporation into *Corynebacterium stationis* CJX1664, which is a strain having 5'-xanthosine monophosphate production ability, and strains in which the mutant gene and the vector were inserted together into the chromosome on a selective medium containing 25 mg/L kanamycin were selected as a primary candidate group. Thereafter, in strains in which homologous recombination had occurred, secondary confirmation was performed by PCR using a primer pair of SEQ ID NO: 3 and SEQ ID NO: 4, followed by final selection of strains into which the mutation was introduced through gene sequence analysis. The selected strain was named CJX1664_purC(H69Q).

### Example 4-2: Evaluation of 5'-xanthosine monophosphate production ability of 5'-xanthosine monophosphate-producing strains into which purC mutations were introduced

In order to measure the XMP production ability of the strain constructed in Example 4-1 above, a flask titer evaluation was performed.

The constructed strain was inoculated into a 14 mL tube containing 2.5 mL of the seed medium described below and subjected to shaking culture at 30°C and 170 rpm for 24 hours. Then, 1 mL of the seed culture was inoculated into a 300 mL corner-baffled flask containing 32 mL of the production medium described below (24 mL of main medium + 8 mL of separately sterilized medium), and shaking culture was performed at 30°C and 170 rpm for 72 hours. After completion of the culture, the XMP production amount was measured using HPLC, and the culture results are shown in Table 7 below.

### <Composition of seed medium for XMP production>

Glucose 1%, peptone 1%, meat extract 1%, yeast extract 1%, sodium chloride 0.25%, adenine 100 mg/L, guanine 100 mg/L, pH 7.5

### <Composition of production medium (main medium) for XMP production>

Glucose 40 g/L, magnesium sulfate 10 g/L, calcium chloride 100 mg/L, ferrous sulfate 20 mg/L, manganese sulfate 10 mg/L, zinc sulfate 10 mg/L, copper sulfate 0.8 mg/L, histidine 20 mg/L, cysteine 15 mg/L, beta-alanine 15 mg/L, biotin 100 µg/L, thiamine 5 mg/L, adenine 50 mg/L, guanine 25 mg/L, niacin 5 mg/L, pH 7.0

### <Composition of production medium (separately sterilized medium) for XMP production>

Monopotassium phosphate18 g/L, dipotassium phosphate 42 g/L, urea 7 g/L, ammonium sulfate 5 g/L

**[Table 7]**

| Confirmation of 5'-xanthosine monophosphate production amount according to introduction of purC gene H69Q mutation | | | | |
|---|---|---|---|---|
| Strain name | Form introduced | OD | 5'-xanthosine monophosphate(g/L) | Concentration increase rate (%) |
| CJX1664 | Control | 54.8 | 4.63 | - |
| CJX1664_purC(H69Q) | purC(H69Q) | 52.7 | 4.83 | 4.3 |

As a result, as shown in Table 7, it was confirmed that the CJX1664_purC(H69Q) strain, into which the purC(H69Q) mutation was introduced into an XMP-producing strain, exhibited an increase of about 4.3% in the concentration of 5'-xanthosine monophosphate as compared with the control CJX1664 strain.

### Example 5: Evaluation of 5'-guanosine monophosphate production ability of strains into which purC mutations were introduced

Using the XMP culture broths of the strains constructed in Example 4-1 above and the control parent strain obtained in Example 4-2 above, 5'-guanosine monophosphate (GMP) production ability was evaluated by the method described below.

Specifically, strains were cultured by the fermentation titer evaluation method of Example 4-2 above, and after completion of the culture, the production amount of XMP (5'-xanthosine monophosphate) was measured using HPLC. In order to convert the produced XMP into GMP, conversion reaction additives below and XMP aminase from *Escherichia coli* were added to the flask fermentation broth, and a conversion reaction was performed at 40°C for 2.5 hours. As a result of performing the above experiment, conversion rate results, which indicate the production amount of GMP relative to the consumption amount of XMP, are shown in Table 8 below.

### <Conversion reaction additives>

Phytic acid 1.8 g/L, magnesium sulfate 4.8 g/L, nymeen 3 ml/L, xylene 2%, adenine 100 mg/L, sodium hydrogen phosphate (Na₂HPO₄) 7.7 g/L, glutamine 2 g/L, glucose 46 g/L

**[Table 8]**

| Confirmation of GMP production ability of a purC(H69Q) mutation-introduced strain | | | | |
|---|---|---|---|---|
| Strain name | Form introduced | 5'-xanthosine monophosphate(g/L) | 5'-guanosine monophosphate(g/L) | Conversion rate (%) (GMP produced/XMP consumed) |
| CJX1664 | Control | 4.53 | 3.25 | 71.9 |
| CJX1664_purC(H69Q) | purC(H69Q) | 4.76 | 3.45 | 72.5 |

As a result, as shown in Table 8, it was confirmed that GMP was produced through a conversion reaction from XMP generated by the strain, and it was confirmed that a greater amount of GMP was produced in the CJX1664_purC(H69Q) strain in which the H69Q mutation was introduced into the purC gene, as compared with the parent strain.

From the above description, those skilled in the art to which the present disclosure pertains will understand that the present disclosure may be embodied in other specific forms without changing the technical spirit or essential features thereof. In this regard, the examples described above are to be understood in all respects as illustrative and not restrictive. The scope of the present disclosure should be construed such that all changes or modified forms derived from the meaning and scope of the claims set forth hereinafter, and equivalents thereof, are included within the scope of the present disclosure, rather than from the above detailed description.

## Claims

1. A polypeptide having phosphoribosyl aminoimidazole-succinocarboxamide synthase activity, in which an amino acid corresponding to the 69th residue from the N-terminus in the amino acid sequence of SEQ ID NO: 5 is substituted with another amino acid.

2. The polypeptide according to claim 1, wherein the amino acid corresponding to the 69th residue from the N-terminus in the amino acid sequence of SEQ ID NO: 5 is substituted with glutamine, alanine, valine, leucine, threonine, asparagine, proline, or serine.

3. The polypeptide according to claim 1, wherein the amino acid corresponding to the 69th residue is histidine.

4. The polypeptide according to claim 1, wherein the polypeptide consists of an amino acid sequence of any one selected from SEQ ID NO: 20 to SEQ ID NO: 27.

5. A polynucleotide encoding the polypeptide of any one claim of claim 1 to claim 4.

6. The polynucleotide according to claim 5, wherein the polynucleotide consists of a nucleic acid sequence of any one selected from SEQ ID NO: 29 to SEQ ID NO: 36.

7. A recombinant vector comprising the polynucleotide of claim 5.

8. A microorganism, comprising at least one selected from the group consisting of the polypeptide of any one claim of claim 1 to claim 4, a polynucleotide encoding the polypeptide, and a vector comprising the polynucleotide.

9. The microorganism according to claim 8, wherein the microorganism has increased purine nucleotide production ability.

10. The microorganism according to claim 9, wherein the purine nucleotide is any one or more selected from the group consisting of 5'-inosine monophosphate (IMP), 5'-xanthosine monophosphate (XMP) and 5'-guanosine monophosphate (GMP).

11. The microorganism according to claim 8, wherein the microorganism is a microorganism of the genus *Corynebacterium*.

12. The microorganism according to claim 11, wherein the microorganism is *Corynebacterium stationis*.

13. A method for producing purine nucleotides, comprising culturing a microorganism comprising at least one selected from the group consisting of the polypeptide of any one claim of claim 1 to claim 4, a polynucleotide encoding the polypeptide, and a vector comprising the polynucleotide in a medium.

14. The method for producing purine nucleotides according to claim 13, further comprising recovering purine nucleotides from the cultured microorganism, medium, or both of them.

15. The method for producing purine nucleotides according to claim 13, wherein the purine nucleotides are any one or more selected from the group consisting of 5'-inosine monophosphate, 5'-xanthosine monophosphate and 5'-guanosine monophosphate.

16. A composition for producing purine nucleotides, comprising a microorganism comprising at least one selected from the group consisting of the polypeptide of any one claim of claim 1 to claim 4, a polynucleotide encoding the polypeptide, and a vector comprising the polynucleotide.
